# EUROPEAN PATENT SPECIFICATION

(11) **EP 3 545 820 B1**
(45) Date of publication and mention of the grant of the patent: **30.07.2025**
(21) Application number: 18164038.4
(22) Date of filing: 26.03.2018
(51) Int. Cl.: G16H 20/40, A61B 5/00, A61B 5/08, A61B 5/087, A61B 5/091, A61B 5/113, G16H 50/20

(54) **RESPIRATORY VOLUME MEASUREMENT**
ATEMVOLUMENMESSUNG
MESURE DE VOLUME RESPIRATOIRE

(43) Date of publication of application: 02.10.2019
(73) Proprietor: Nokia Technologies Oy, 02610 Espoo (FI)
(72) Inventor: MURGAS, Matej, 00240 Helsinki (FI); RAJALA, Satu, 36200 Kangasala (FI)
(74) Representative: Swindell & Pearson Limited

(56) References cited:
- US-A1- 2014 228 657
- JOHN DINESH ET AL: "Simple to complex modeling of breathing volume using a motion sensor", SCIENCE OF THE TOTAL ENVIRONMENT, ELSEVIER, AMSTERDAM, NL, vol. 454, 27 March 2013 (2013-03-27), pages 184 - 188, XP028589595, ISSN: 0048-9697, DOI: 10.1016/J.SCITOTENV.2013.02.092
- NGUYEN PHUC ET AL: "Continuous and fine-grained breathing volume monitoring from afar using wireless signals", IEEE INFOCOM 2016 - THE 35TH ANNUAL IEEE INTERNATIONAL CONFERENCE ON COMPUTER COMMUNICATIONS, IEEE, 10 April 2016 (2016-04-10), pages 1 - 9, XP032929994, DOI: 10.1109/INFOCOM.2016.7524402
- DUMOND RÉMY ET AL: "Estimation of respiratory volume from thoracoabdominal breathing distances: comparison of two models of machine learning", EUROPEAN JOURNAL OF APPLIED PHYSIOLOGY, SPRINGER VERLAG, HEIDELBERG, DE, vol. 117, no. 8, 13 June 2017 (2017-06-13), pages 1533 - 1555, XP036274169, ISSN: 1439-6319, [retrieved on 20170613], DOI: 10.1007/S00421-017-3630-0

## Description

### TECHNOLOGICAL FIELD

Embodiments relate to respiratory volume measurement. Some embodiments relate to an apparatus, others to a method and others to a computer program.

### BACKGROUND

It may be desirable to measure respiration of a subject, for example a human or another animal with lungs, that respires by inhaling and exhaling. When the subject inhales the lungs fill with air and the chest wall moves outwards and when the subject exhales the lungs deflate and the chest wall moves outwards.

US2014/0228657 discloses a system and method for monitoring respiration, comprising a respiration sensing module 110 and a supplementary sensing module 120. The system is configured to be fixed to a subject's body (for example, the torso area).

The motion sensor types used in the respiration sensing module may include a force sensing resistor, a capacitor, a hall-effect probe, a piezoelectric sensor, a microphone and an ultrasonic sensor. The supplementary sensing module uses an accelerometer to distinguish between overall body motion of a subject and motion due to a subject's respiration.

Technical paper "Simple to complex modelling of breathing volume using a motion sensor", discloses that motion sensors such as accelerometers may be useful to estimate VE (ventilation).

Both documents disclose the application of machine learning when monitoring respiration.

### BRIEF SUMMARY

The invention is defined by the appended claims.

According to a first aspect, it is provided a respiratory volume measurement system comprising means for: receiving at least one accelerometer sensor output signal dependent upon respiratory motion of a chest wall of a subject; and producing a respiration measurement output, different to the at least one accelerometer sensor output signal, that provides a measure of respiration volume of the subject, wherein the respiration measurement output is produced using machine learning, and takes as input the time differential of the accelerometer sensor output signal.

According to a second aspect, it is provided a computer implemented method comprising:receiving an accelerometer sensor output signal dependent upon respiratory motion of a chest wall of a subject; and producing a respiration measurement output, different to the accelerometer sensor output signal, that provides a measure of respiration volume of the subject, wherein the respiration measurement output is produced using machine learning, and takes as input the time differential of the accelerometer sensor output signal.

According to a third aspect, it is provided a computer program comprising instructions that when executed by a processor cause determination, based on machine learning, of a respiration measurement that provides a measure of respiration volume of a subject using as input the time differential of a received accelerometer sensor output signal dependent upon respiratory motion of a chest wall of the subject.

Preferred embodiments are defined by the dependent claims.

### BRIEF DESCRIPTION

Some example embodiments will now be described with reference to the accompanying drawings in which:
FIG. 1 shows an example embodiment of the subject matter described herein;
FIG. 2 shows another example embodiment of the subject matter described herein;
FIG. 3 shows an example embodiment of the subject matter described herein;
FIG. 4A shows an example embodiment of the subject matter described herein;
FIG. 4B shows an example embodiment of the subject matter described herein;
FIG. 5 shows an example embodiment of the subject matter described herein;
FIG. 6A shows an example embodiment of the subject matter described herein;
FIG. 6B shows an example embodiment of the subject matter described herein;
FIG. 7A shows an example embodiment of the subject matter described herein;
FIG. 7B shows an example embodiment of the subject matter described herein;
FIG. 8 shows an example embodiment of the subject matter described herein;
FIG. 9 shows an example embodiment of the subject matter described herein;
FIG. 10A shows an example embodiment of the subject matter described herein;
FIG. 10B shows an example embodiment of the subject matter described herein;
FIG. 11A shows an example embodiment of the subject matter described herein;
FIG. 11B shows an example embodiment of the subject matter described herein;
FIG. 11C shows an example embodiment of the subject matter described herein.

### DETAILED DESCRIPTION

It would be desirable to measure respiration of a subject by measuring motion of a chest wall of a subject. The subject may be a human or an animal.

Motion of the chest wall of a subject may, for example, be measured by attaching a motion sensor to the subject's chest wall. The sensor then produces an output signal dependent upon the respiratory motion of the chest wall of the subject. Subsequent processing of the sensor output signal can then be used to produce a respiration measurement output that provides a measure of respiration volume of the subject.

A measure of respiration volume is a value that is dependent upon respiration volume and from which a value for respiration volume can be obtained. For example, the respiration volume is a measure of the respiration volume. For example, a respiration flow-rate is a measure of the respiration volume as it can be integrated to obtain the respiration volume. Therefore, subsequent processing of the sensor output signal can then be used to produce a respiration measurement output that provides a respiration volume of the subject. Therefore, subsequent processing of the sensor output signal can then be used to produce a respiration measurement output that provides a respiration flow rate of the subject.

Other parameters may also be measured such as inspiration/expiration phase start, duration of inspiration phase, duration of expiration phase, duration of respiration cycle.

Motion of the chest wall of a subject may, for example, be measured by attaching multiple motion sensors to the subject's chest wall. Each sensor then produces an output signal dependent upon the respiratory motion of the chest wall of the subject. Subsequent processing of the sensor output signals can then be used to produce a respiration measurement output that provides a measure of respiration volume of the subject.

In the example described below, a respiratory volume measurement system 100 uses a machine learning system 20 to process a sensor output signal 11 or to process sensor output signals 11. The machine learning system 20 produces as a respiration measurement output a measure of respiration volume of a subject 30. It can also provide a measure of respiration rate of the subject 30.

A measure of respiration volume is a value that is dependent upon respiration volume and from which a value for respiration volume can be obtained. For example, the respiration volume is a measure of the respiration volume. For example, a respiration flow-rate is a measure of the respiration volume as it can be integrated to obtain the respiration volume. Therefore, in some examples, the machine learning system 20 produces, as a respiration measurement output, a respiration volume of the subject. Therefore, in some examples, additionally or alternatively, the machine learning system 20 produces, as a respiration measurement output, a respiration flow rate of the subject. Other parameters may also be measured such as inspiration/expiration phase start, duration of inspiration phase, duration of expiration phase, duration of respiration cycle.

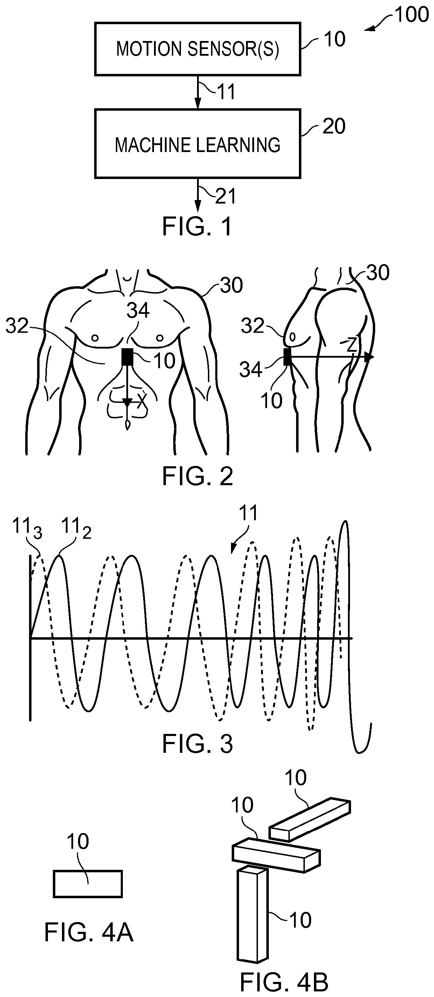

For example, Fig. 1 illustrates an example of a respiratory volume measurement system 100 comprising one or more sensors 10 and a machine learning system 20. At least one of the motion sensors is configured to be placed on a chest wall 32 of the subject 30 (see Fig. 2) to produce a sensor output signal 11 dependent upon respiratory motion of the chest wall 32 of the subject 30. If there is more than one motion sensor 10 then each of the motion sensors 10 may be configured to be placed on the chest wall 32 of the subject 30 to produce a respective sensor output signal 11 dependent upon respiratory motion of the chest wall 32 of the subject 30.

The machine learning system 20 is configured to receive the sensor output signal 11 (or sensor output signals 11) as an input and to produce a respiration measurement output 21, different to the sensor output signal 11, that provides a measure of respiration volume of the subject 30.

The respiratory measurement output 21 quantifies the respiration volume of the subject 30. This quantification may, for example, be relative to the subject 30 measuring for example a percentage increase or decrease relative to a previously determined respiration volume of the subject. Alternatively, the respiratory measurement output 21 may quantify the respiration volume of the subject relative to an absolute volume scale, for example ml (milliliters) or ml/s (milliliters per second). The term 'absolute' implies measurement against a reference scale rather than a relative measurement. It does not necessarily imply a higher accuracy of measurement.

Fig. 2 illustrates an example of a subject 30. In this example a motion sensor 10 is configured to be positioned and held at a sternum 34 of the human subject 30 and to move with respiratory motion of the chest wall 32. In this example, the motion sensor 10 is configured to measure both dorsal-ventral movement (anterior-posterior movement) as indicated by the z-axis ( reference z in the Fig) and also to measure longitudinal measurement as indicated by the x-axis (reference x in the Fig). The direction of longitudinal movement is head-toe and is orthogonal to the dorsal-ventral movement. In other examples only dorsal-ventral movement may be measured or only longitudinal movement may be measured. The one or more sensors 10 may be held in position at the sternum 34 by a belt (not illustrated for clarity).

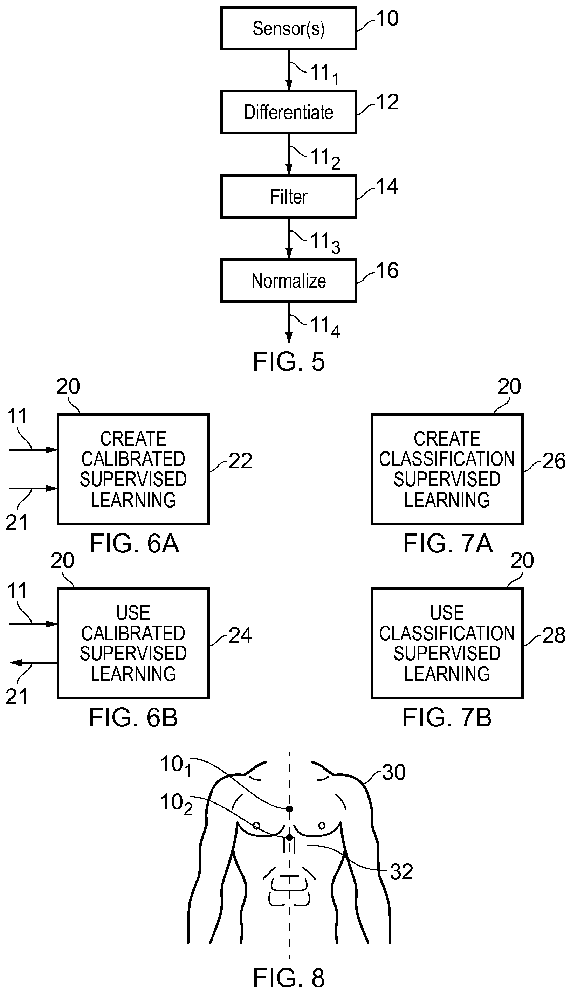

The sensor output signal 11 from a sensor may be post-processed by the sensor 10 or pre-processed by the machine learning system 20 or elsewhere, to produce a new sensor output signal 11. This processing will be described in further detail in relation to Fig. 5. However, it may for example be desirable to differentiate a sensor output signal 11 that represents acceleration in the dorsal-ventral direction and/or the longitudinal direction. Fig. 3 illustrates an example in which the sensor output signal 11₂ represents a time-variable acceleration signal and the sensor output signal 11₃ represents the first-order time-differential of that signal. One or both of the sensor output signals 11₂, 11₃ may for example be used by the machine learning system 20 to obtain the respiration measurement output 21.

Fig. 4A illustrates an example of a motion sensor 10 that comprises one or more sensors for measuring motion. The motion may, for example, be a time-variable displacement (linear and/or angular), or a first or second derivative thereof, measured relative to one, two or three orthogonal axes. The motion sensor 10 may, for example, measure the time-variable displacement (linear and/or angular) directly or measure a force that depends upon the time-variable displacement (linear and/or angular) and which can be converted to the time-variable displacement (linear and/or angular).

For example, a linear motion sensor, for example an accelerometer 10 can measure linear acceleration and produce a sensor output signal 11 that is proportional to acceleration in a particular direction.

For example, an angular motion sensor, for example an accelerometer 10 can measure angular acceleration (rate of change of angular velocity) and produce a sensor output signal 11 that is proportional to acceleration about a particular axis.

For example, an angular motion sensor, for example a gyroscope 10 can measure orientation or angular velocity about a particular axis.

The axis of an accelerometer 10 is therefore defined, for a linear accelerometer, as the direction of linear acceleration that is measured and for a rotational accelerometer or gyroscope , as the axis of rotation about which rotation is measured.

A multiple axis accelerometer measures acceleration in relation to multiple orthogonal axes.

Fig. 4B illustrates that multiple motion sensors 10 may be aligned along multiple different orthogonal axes to form a multiple axis motion sensor. In this example a three-axis system is described. For example, one of the axis can be aligned with the dorsal-ventral (anterior-posterior) direction of the subject 30, another axis can be aligned with a lateral direction of the subject 30 and another axis can be aligned with a longitudinal direction.

In some examples only linear motion sensors 10 (e.g. accelerometers) may be used, in other examples only angular motion sensors 10( e.g. angular accelerometers or gyroscopes) may be used and in other examples both linear and angular accelerometers may be used. One advantage of using angular accelerometers or gyroscopes is that they are not susceptible or are less susceptible to the effects of gravity. The effects of gravity may produce an error in the operation of linear accelerometers. However, gravity is constant and this constant error can be removed by differentiation of the sensor output signal 11.

Small scale linear accelerometers and angular accelerometers or gyroscopes are used in mobile electronic devices such as mobile telephones and similar sensors can be used as sensors 10.

A single device may be used as a linear accelerometer and an angular accelerometer or gyroscope.

Fig. 5 illustrates an example of processing of a sensor output signal 11 before it is processed by an algorithm of the machine learning system 20. This processing may be performed as post-processing at the sensor 10 or as pre-processing at the machine learning system 20 or elsewhere.

The motion sensor 10 produces a sensor output signal 11₁. The sensor output signal 11₁ is differentiated by the differentiator 14 to produce sensor output signal 11₂ The sensor output signal 11₂ is filtered by the filter 12 to produce sensor output signal 11₃. and the sensor output signal 11₃ is normalized by normalizer 16 to produce the sensor output signal 11₄ which is used by the machine learning system 20.

It should be appreciated that the filter 12, the differentiator 14 and the normalizer 16 are optional and any one of them, or any combination of them may or may not be used. It should also be appreciated that they may perform their functionality in the analogue domain or in the digital domain.

It should be appreciated that the filter 12 and/or the differentiator 14 and/or the normalizer 16 can implemented as circuitry or as software or firmware performed by a processor.

The filter 12 may be a low pass filter that passes signals with a frequency of less than 1 Hz or substantially 1Hz or lower or higher.

DC signals from the sensor 10 do not represent changes in the respiratory volume. Therefore, in some examples, the filter 12 may be a band-pass filter with a range greater than 0.05 Hz or substantially 0.05Hz. For example, it may be a band-pass filter with low attenuation in the range 0.05 Hz to 1 Hz or substantially in the range 0.05 Hz to 1 Hz.

In some examples, the filter 12 may be an adaptive filter that varies. For example, it may be configured to change its band-pass in dependence upon a heart rate of the subject or increasing frequency of respiration so that the filter 12 can accommodate normal respiratory conditions at rest and also normal respiratory conditions during exercise.

The differentiator 14 is configured to receive the sensor output signal 11₂ as an input and to differentiate the sensor output signal 11₂ to produce an input parameter for the machine learning algorithm. In the example illustrated it produces the sensor output signal 11₃. The differentiation may be a first order time differentiation or it may be higher order differentiation. In the example where the sensor output signal 11₁ represents acceleration measured by an accelerometer 10 then the first differential in time produced by the differentiator 14 represents a jerk (the rate of change of acceleration) and this is output as the sensor output signal 11₃.

The normalizer 16 is configured to receive a sensor output signal 11₃ and normalize the signal to produce a sensor output signal 11₄. This may be achieved by dividing the signal 11₃ by its maximum value and processing it so that it has a zero mean.

Although Fig. 5 illustrates only a single sensor 10, it should be appreciated that a similar process may be performed for each sensor 10 where there are multiple sensors 10. The outcome of the process illustrated in Fig. 5 is the production of an input parameter p for a machine learning algorithm used by the machine learning system 20. Where multiple sensors 10ᵢ are used the input parameters pᵢ may be combined in different weighted combinations of the parameters and/or functions of the parameters either as pre-processing for input to the machine learning algorithm or as part of the machine learning algorithm. The parameters or combined parameters are, however, still sensor output signals 11.

Figs 6A and 6B illustrate the use of the machine learning system 20 to provide an absolute measurement of the respiration volume of the subject by calibration.

Fig. 6A illustrates the training process that calibrate the system. During the calibration process various sensor output signals 11 are provided to the machine learning algorithm along with corresponding absolute values of respiration volume as training data. The machine learning algorithm generates a function that takes as inputs the various sensor output signals 11 and produces as outputs correct estimates of the corresponding absolute values of respiration volume. This may be achieved using supervised learning using a suitable machine learning algorithm such as linear regression, quadratic regression, neural networks with back propagation.

Once the machine learning algorithm has been trained using the above-described supervised learning process, as illustrated in Fig 6B, the calibrated machine learning system 20 is able to receive as an input a sensor output signal 11 and to produce automatically a respiration measurement output 21 that provides an absolute measurement of the respiration volume of the subject 30.

Figs 7A and 7B illustrate the use of the machine learning system 20 to identify a respiration pattern of the subject by classification.

Fig. 7A illustrates the training process. During the training process various sensor output signals 11 are provided to the machine learning algorithm. The machine learning algorithm generates a function that takes as inputs the various sensor output signals 11 and classifies the inputs at that time into one or multiple different classes. This may be achieved using unsupervised learning using a suitable machine learning algorithm such as logistic regression.

Once the machine learning algorithm has been trained using the above-described unsupervised learning process, as illustrated in Fig 7B, the trained machine learning system 20 is able to receive as an input a sensor output signal 11 and to produce automatically a respiration measurement output 21 that classifies the input.

The trained machine learning system 20 can classify breathing patterns and identify breathing patterns that deviate from a reference breathing pattern(s) using the respiration measurement output 21. It can, for example, detect a breathing pattern produced by sleep apnoea (also spelt apnea)

In the example illustrated in Fig. 8, multiple motion sensors 10 are used and they are deliberately physically separated from each other. The purpose of this separation is so that they are both affected by gross movements of the subject 30 to the same or substantially the same extent but are subjected to different movements in response to movement of the chest wall 32 caused by respiration of the subject 30. The difference between the sensor output signals 11 from the positionally offset sensors 10 is therefore substantially independent of the gross motion of the subject 30 and is dependent upon the motion of the chest wall 32 of the subject 30 as a consequence of respiration. This difference signal may therefore be used as a sensor output signal 11 provided to the machine learning system 20. In this way, the effect of gross motion of the subject's body may be mitigated.

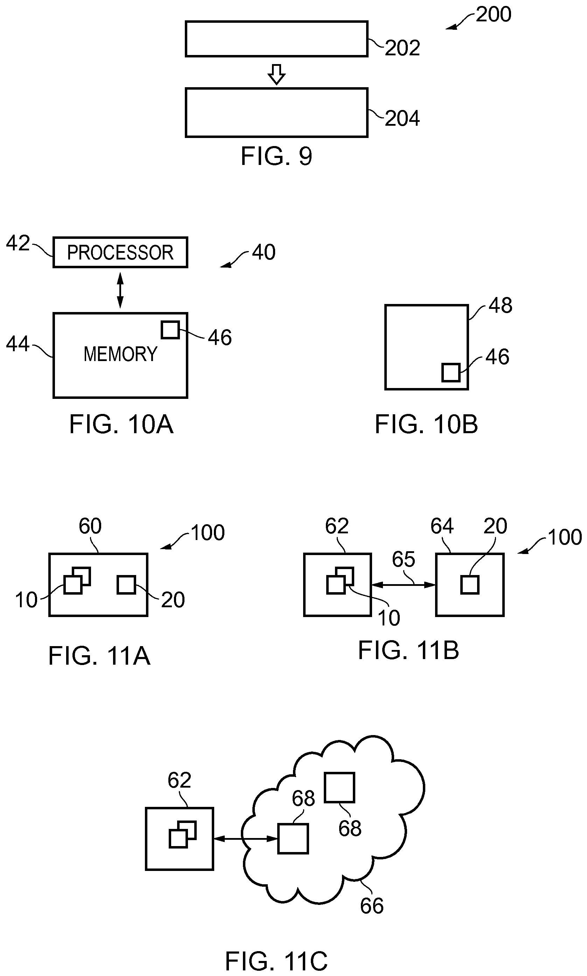

Fig. 9 illustrates an example of a method 200.

At block 202, the method 200 comprises receiving a sensor output signal 11 dependent upon respiratory motion of a chest wall 32 of a subject 30.

At block 204, the method 200 comprises producing a respiration measurement output 21, different to the sensor output signal 11, that provides a measurement of respiration volume of the subject 30, wherein the respiration measurement output 21 is produced using machine learning.

As described before in relation to the preceding examples, the method 200 may quantify the respiration volume of the subject 30 using calibration or classification.

Fig 10A illustrates an example of a controller 40. Implementation of a controller 40 may be as controller circuitry. The controller 40 may be implemented in hardware alone, have certain aspects in software including firmware alone or can be a combination of hardware and software (including firmware).

As illustrated in Fig 10A the controller 40 may be implemented using instructions that enable hardware functionality, for example, by using executable instructions of a computer program 46 in a general-purpose or special-purpose processor 42 that may be stored on a computer readable storage medium (disk, memory etc) to be executed by such a processor 42.

The processor 42 is configured to read from and write to the memory 44. The processor 42 may also comprise an output interface via which data and/or commands are output by the processor 42 and an input interface via which data and/or commands are input to the processor 42.

The memory 44 stores a computer program 46 comprising computer program instructions (computer program code) that controls the operation of the machine learning system 20 when loaded into the processor 42. The computer program instructions, of the computer program 46, provide the logic and routines that enables the apparatus to perform the methods illustrated in Fig. 9. The processor 42 by reading the memory 44 is able to load and execute the computer program 46.

The machine learning system 20 therefore comprises:
at least one processor 42; and
at least one memory 44 including computer program code
the at least one memory 44 and the computer program code configured to, with the at least one processor 42, cause the machine learning system 20 at least to perform: determining, based on machine learning, of a respiration measurement that provides a measure of respiration volume of a subject 30 using a received sensor output signal 11 dependent upon respiratory motion of a chest wall 32 of the subject 30.

Quantification of the respiration volume of the subject 30 uses, for example, calibration or classification.

As illustrated in Fig 10B, the computer program 46 may arrive at the machine learning system 20 via any suitable delivery mechanism 48. The delivery mechanism 48 may be, for example, a machine readable medium, a computer-readable medium, a non-transitory computer-readable storage medium, a computer program product, a memory device, a record medium such as a Compact Disc Read-Only Memory (CD-ROM) or a Digital Versatile Disc (DVD) or a solid state memory, an article of manufacture that comprises or tangibly embodies the computer program 46. The delivery mechanism may be a signal configured to reliably transfer the computer program 46. The machine learning system 20 may propagate or transmit the computer program 46 as a computer data signal.

The computer program 46 comprises instructions that cause determination, based on machine learning, of a respiration measurement that provides a measure of respiration volume of a subject 30 using a received sensor output signal 11 dependent upon respiratory motion of a chest wall 32 of the subject 30. The instructions cause quantification of the respiration volume of the subject 30 using, for example, calibration or classification.

The computer program instructions may be comprised in a computer program, a non-transitory computer readable medium, a computer program product, a machine readable medium. In some but not necessarily all examples, the computer program instructions may be distributed over more than one computer program.

Although the memory 44 is illustrated as a single component/circuitry it may be implemented as one or more separate components/circuitry some or all of which may be integrated/removable and/or may provide permanent/semi-permanent/ dynamic/cached storage.

Although the processor 42 is illustrated as a single component/circuitry it may be implemented as one or more separate components/circuitry some or all of which may be integrated/removable. The processor 42 may be a single core or multi-core processor.

References to 'computer-readable storage medium', 'computer program product', 'tangibly embodied computer program' etc. or a 'controller', 'computer', 'processor' etc. should be understood to encompass not only computers having different architectures such as single /multi- processor architectures and sequential (Von Neumann)/parallel architectures but also specialized circuits such as field-programmable gate arrays (FPGA), application specific circuits (ASIC), signal processing devices and other processing circuitry. References to computer program, instructions, code etc. should be understood to encompass software for a programmable processor or firmware such as, for example, the programmable content of a hardware device whether instructions for a processor, or configuration settings for a fixed-function device, gate array or programmable logic device etc.

As used in this application, the term 'circuitry' may refer to one or more or all of the following:
(a) hardware-only circuitry implementations (such as implementations in only analog and/or digital circuitry) and
(b) combinations of hardware circuits and software, such as (as applicable):
   (i) a combination of analog and/or digital hardware circuit(s) with software/firmware and
   (ii) any portions of hardware processor(s) with software (including digital signal processor(s)), software, and memory(ies) that work together to cause an apparatus, such as a mobile phone or server, to perform various functions and
(c) hardware circuit(s) and or processor(s), such as a microprocessor(s) or a portion of a microprocessor(s), that requires software (e.g. firmware) for operation, but the software may not be present when it is not needed for operation.

This definition of circuitry applies to all uses of this term in this application, including in any claims. As a further example, as used in this application, the term circuitry also covers an implementation of merely a hardware circuit or processor and its (or their) accompanying software and/or firmware. The term circuitry also covers, for example and if applicable to the particular claim element, a baseband integrated circuit for a mobile device or a similar integrated circuit in a server, a cellular network device, or other computing or network device.

The blocks illustrated in the Fig. 9 may represent steps in a method and/or sections of code in the computer program 46. The illustration of a particular order to the blocks does not necessarily imply that there is a required or preferred order for the blocks and the order and arrangement of the block may be varied. Furthermore, it may be possible for some blocks to be omitted.

Figs. 11A, 11B and 11C illustrate different examples of implementation of the respiratory volume measurement system 100.

In the example illustrated in Fig. 11A the respiratory volume measurement system 100 is housed entirely within a single apparatus 60 which comprises one or more motion sensors 10 and the machine learning system 20. In this example the apparatus 60 may be affixed to the chest wall of the subject 30. The apparatus 60 may have communication means for communicating in a network (not illustrated).

Fig. 11B illustrates that the respiratory volume measurement system 100 may be split into two different apparatus 62, 64. In this example the apparatus 62 comprises one or more motion sensors 10 and the apparatus 64 comprises the machine learning system 20. Both the one or more sensors 10 and the machine learning system 20 are able to communicate between the apparatus 62 and the apparatus 64 via a communication channel 65. This communication channel may, for example, be provided via a wireless interface. In this example the apparatus 62 may be placed upon the chest wall of the user. The apparatus 64 may be local but separated from the apparatus 62 or it may be separated at some distance from the apparatus 62 via a network, for example.

Fig. 11C illustrates a development of the system 100 illustrated in Fig. 11B. In this example the apparatus 64 is replaced by a distributed network 66 comprising multiple apparatus 68.

It should be appreciated that the production of the sensor output signal(s) 11 and the production of the respiration measurement output 21 may be separated in time and/or space. For example, the machine learning system 20 may receive the sensor output signal 11 live and produce, in real time, a respiration measurement output 21. However, in other examples the sensor output signals 11 may be stored in a memory system and the machine learning system 20 may at a later time process those signals to produce the respiration measurement output 21.

Also, as previously described, the motion sensor 10 and the machine learning system may be separated in space or may be in the same location or apparatus.

In the description, features are illustrated as coupled. These features are operationally coupled and any number or combination of intervening elements can exist (including no intervening elements)

Where a structural feature has been described, it may be replaced by means for performing one or more of the functions of the structural feature whether that function or those functions are explicitly or implicitly described.

The controller 40 performs the function of the machine learning system 20 and may be replaced by any suitable process means. It may, for example, be a programmable processor controlled by software or firmware, circuitry, programmed logic gates, multiple processors.

The respiratory volume measurement system 100 comprising means for: receiving at sensor output signal 11 dependent upon respiratory motion of a chest wall 32 of a subject 30; and for producing a respiration measurement output 21, different to the sensor output signal 11, that provides a measure of respiration volume of the subject 30, wherein the respiration measurement output 21 is produced using machine learning.

The respiration volume of the subject 30 may, for example, be quantified using calibration or classification.

In some but not necessarily all examples, the machine learning system 20 is configured to communicate data from the machine learning system 20 with or without local storage of the data in a memory 44 at the machine learning system 20 and with or without local processing of the data by circuitry or processors at the machine learning system 20.

The data may, for example, be measurement data from the sensors 10 or data produced by the processing of measurement data from the machine learning system 20.

The data may be stored in processed or unprocessed format remotely at one or more devices. The data may be stored in a cloud-based storage system.

The data may be processed remotely at one or more devices. The data may be partially processed locally and partially processed remotely at one or more devices.

The data may be communicated to the remote devices wirelessly via short range radio communications such as Wi-Fi or Bluetooth, for example, or over long range cellular radio links. The apparatus may comprise a communications interface such as, for example, a radio transceiver for communication of data.

The machine learning system 20 may be part of the Internet of Things forming part of a larger, distributed network.

The processing of the data, whether local or remote, may be for the purpose of health monitoring, data aggregation, patient monitoring, vital signs monitoring or other purposes.

The processing of the data, whether local or remote, may involve artificial intelligence or machine learning algorithms. The data may, for example, be used as learning input to train a machine learning network or may be used as a query input to a machine learning network, which provides a response. The machine learning network may for example use linear regression, logistic regression, vector support machines or an acyclic machine learning network such as a single or multi hidden layer neural network.

The processing of the data, whether local or remote, may produce an output. The output may be communicated to the machine learning system 20 where it may produce an output sensible to the subject such as an audio output, visual output or haptic output.

The systems, apparatus, methods and computer programs may use machine learning which can include statistical learning. Machine learning is a field of computer science that gives computers the ability to learn without being explicitly programmed. The computer learns from experience E with respect to some class of tasks T and performance measure P if its performance at tasks in T, as measured by P, improves with experience E. The computer can often learn from prior training data to make predictions on future data. Machine learning includes wholly or partially supervised learning and wholly or partially unsupervised learning. It may enable discrete outputs (for example classification, clustering) and continuous outputs (for example regression). Machine learning may for example be implemented using different approaches such as cost function minimization, artificial neural networks, support vector machines and Bayesian networks for example. Cost function minimization may, for example, be used in linear and polynomial regression and K-means clustering. Artificial neural networks, for example with one or more hidden layers, model complex relationship between input vectors and output vectors. Support vector machines may be used for supervised learning. A Bayesian network is a directed acyclic graph that represents the conditional independence of a number of random variables.

The algorithms hereinbefore described may be applied to achieve the following technical effects: measurement of respiration volume of the subject. quantification of respiration volume of the subject, an absolute measurement of respiration volume of the subject, detection of breathing patterns of a subject.

The above described examples may find application as enabling components of:
automotive systems; telecommunication systems; electronic systems including consumer electronic products; distributed computing systems; media systems for generating or rendering media content including audio, visual and audio visual content and mixed, mediated, virtual and/or augmented reality; personal systems including personal health systems or personal fitness systems; navigation systems; user interfaces also known as human machine interfaces; networks including cellular, non-cellular, and optical networks; ad-hoc networks; the internet; the internet of things; virtualized networks; and related software and services.

The term 'comprise' is used in this document with an inclusive not an exclusive meaning. That is any reference to X comprising Y indicates that X may comprise only one Y or may comprise more than one Y. If it is intended to use 'comprise' with an exclusive meaning then it will be made clear in the context by referring to "comprising only one.." or by using "consisting".

In this description, reference has been made to various examples. The description of features or functions in relation to an example indicates that those features or functions are present in that example. The use of the term 'example' or 'for example' or 'can' or 'may' in the text denotes, whether explicitly stated or not, that such features or functions are present in at least the described example, whether described as an example or not, and that they can be, but are not necessarily, present in some of or all other examples. Thus 'example', 'for example', 'can' or 'may' refers to a particular instance in a class of examples. A property of the instance can be a property of only that instance or a property of the class or a property of a sub-class of the class that includes some but not all of the instances in the class. It is therefore implicitly disclosed that a feature described with reference to one example but not with reference to another example, can where possible be used in that other example as part of a working combination but does not necessarily have to be used in that other example. Although embodiments have been described in the preceding paragraphs with reference to various examples, it should be appreciated that modifications to the examples given can be made without departing from the scope of the claims.

Features described in the preceding description may be used in combinations other than the combinations explicitly described above.

Although functions have been described with reference to certain features, those functions may be performable by other features whether described or not.

Although features have been described with reference to certain embodiments, those features may also be present in other embodiments whether described or not.

The term 'a' or 'the' is used in this document with an inclusive not an exclusive meaning. That is any reference to X comprising a/the Y indicates that X may comprise only one Y or may comprise more than one Y uncles the context clearly indicates the contrary. If it is intended to use 'a' or 'the' with an exclusive meaning then it will be made clear in the context. In some circumstances the use of 'at least one' or 'one or more' may be used to emphasis an inclusive meaning but the absence of these terms should not be taken to infer and exclusive meaning.

In this description, reference has been made to various examples using adjectives or adjectival phrases to describe characteristics of the examples. Such a description of a characteristic in relation to an example indicates that the characteristic is present in some examples exactly as described and is present in other examples substantially as described.

The use of the term 'example' or 'for example' or 'can' or 'may' in the text denotes, whether explicitly stated or not, that such features or functions are present in at least the described example, whether described as an example or not, and that they can be, but are not necessarily, present in some of or all other examples. Thus 'example', for example', 'can' or 'may' refers to a particular instance in a class of examples. A property of the instance can be a property of only that instance or a property of the class or a property of a sub-class of the class that includes some but not all of the instances in the class. It is therefore implicitly disclosed that a feature described with reference to one example but not with reference to another example, can where possible be used in that other example as part of a working combination but does not necessarily have to be used in that other example.

The invention is defined by the appended claims.

## Claims

1. A respiratory volume measurement system (100) comprising means for:
receiving at least one accelerometer sensor output signal (11) dependent upon respiratory motion of a chest wall (32) of a subject (30); and
producing a respiration measurement output (21), different to the at least one accelerometer sensor output signal, that provides a measure of respiration volume of the subject, wherein the respiration measurement output is produced using machine learning, and takes as input the time differential of the accelerometer sensor output signal.

2. A respiratory volume measurement system (100) as claimed in claim 1 comprising means configured to quantify the respiration volume of the subject using calibration or classification.

3. A respiratory volume measurement system (100) as claimed in claim 1 or 2, comprising means configured to perform supervised learning to calibrate the produced respiration measurement output for the subject.

4. A respiratory volume measurement system (100) as claimed in claim 1 or 2, comprising means configured to classify breathing patterns.

5. A respiratory volume measurement system (100) as claimed in claim 1, 2 or 4 comprising means configured to detect sleep apnoea.

6. A respiratory volume measurement system (100) as claimed in any preceding claim wherein the at least one accelerometer sensor output signal (11) comprises at least one linear motion signal and/or one or more angular motion signals.

7. A respiratory volume measurement system (100) as claimed in any preceding claim comprising means configured to receive multiple sensor output signals as inputs and to produce a respiration measurement output that provides a measure of respiration volume of the subject in dependence upon different combinations and/or weightings of the multiple sensor output signals.

8. A respiratory volume measurement system (100) as claimed in any preceding claim comprising means configured to normalize the accelerometer sensor output signal (11) to produce an input parameter for a machine learning algorithm.

9. A respiratory volume measurement system (100) as claimed in any preceding claim comprising means configured to receive and use accelerometer sensor output signals from multiple motion sensors (10) at different positions to remove or mitigate motion artefacts arising from motion of the subject (30) other than motion of the chest wall (32) of the subject (30).

10. A health monitoring system comprising the respiratory measurement system as claimed in any preceding claim.

11. A computer implemented method comprising:
receiving an accelerometer sensor output signal (11) dependent upon respiratory motion of a chest wall (32) of a subject (30); and
producing a respiration measurement output, different to the accelerometer sensor output signal, that provides a measure of respiration volume of the subject, wherein the respiration measurement output is produced using machine learning, and takes as input the time differential of the accelerometer sensor output signal.

12. A method as claimed in claim 11 comprising:
quantifying the respiration volume of the subject using calibration or classification.

13. A computer program (46) comprising instructions that when executed by a processor cause determination, based on machine learning, of a respiration measurement that provides a measure of respiration volume of a subject using as input the time differential of a received accelerometer sensor output signal (11) dependent upon respiratory motion of a chest wall (32) of the subject (30).

14. A computer program (46) as claimed in claim 13 wherein the instructions cause quantification of the respiration volume of the subject using calibration or classification.

## Patentansprüche

1. Atemvolumen-Messsystem (100), umfassend Mittel zum:
Empfangen mindestens eines Beschleunigungssensor-Ausgangssignals (11), das von der Atembewegung einer Brustwand (32) einer Person (30) abhängt; und
Erzeugen einer Atmungsmessungsausgabe (21), die sich von dem mindestens einen Beschleunigungssensor-Ausgangssignal unterscheidet und ein Maß für das Atemvolumen der Person bereitstellt, wobei die Atmungsmessungsausgabe unter Verwendung von maschinellem Lernen erzeugt wird und als Eingabe das Zeitdifferential des Beschleunigungssensor-Ausgangssignals verwendet.

2. Atemvolumen-Messsystem (100) nach Anspruch 1, das Mittel umfasst, die so konfiguriert sind, dass sie das Atemvolumen der Person unter Verwendung von Kalibrierung oder Klassifizierung quantifizieren.

3. Atemvolumen-Messsystem (100) nach Anspruch 1 oder 2, das Mittel umfasst, die so konfiguriert sind, dass sie überwachtes Lernen durchführen, um die erzeugte Atmungsmessungsausgabe für die Person zu kalibrieren.

4. Atemvolumen-Messsystem (100) nach Anspruch 1 oder 2, das Mittel umfasst, die so konfiguriert sind, dass sie Atmungsmuster klassifizieren.

5. Atemvolumen-Messsystem (100) nach Anspruch 1, 2 oder 4, das Mittel umfasst, die so konfiguriert sind, dass sie Schlafapnoe erkennen.

6. Atemvolumen-Messsystem (100) nach einem der vorstehenden Ansprüche, wobei das mindestens eine Beschleunigungssensor-Ausgangssignal (11) mindestens ein lineares Bewegungssignal und/oder ein oder mehrere Winkelbewegungssignale umfasst.

7. Atemvolumen-Messsystem (100) nach einem der vorstehenden Ansprüche, das Mittel umfasst, die so konfiguriert ist, dass sie mehrere Sensorausgangssignale als Eingaben empfangen und eine Atmungsmessungsausgabe erzeugen, die ein Maß für das Atemvolumen der Person in Abhängigkeit von verschiedenen Kombinationen und/oder Gewichtungen der mehreren Sensorausgangssignale liefert.

8. Atemvolumen-Messsystem (100) nach einem der vorstehenden Ansprüche, das Mittel umfasst, die so konfiguriert sind, dass sie das Beschleunigungssensor-Ausgangssignal (11) normalisieren, um einen Eingabeparameter für einen maschinellen Lernalgorithmus zu erzeugen.

9. Atemvolumen-Messsystem (100) nach einem der vorstehenden Ansprüche, das Mittel umfasst, die so konfiguriert ist, dass sie Beschleunigungssensor-Ausgangssignale von mehreren Bewegungssensoren (10) an verschiedenen Positionen empfangen und verwenden, um Bewegungsartefakte zu entfernen oder abzuschwächen, die durch eine andere Bewegung der Person (30) als die Bewegung der Brustwand (32) der Person (30) entstehen.

10. Gesundheitsüberwachungssystem, das das in einem der vorstehenden Ansprüche beanspruchte Atmungsmesssystem umfasst.

11. Computerimplementiertes Verfahren, umfassend:
Empfangen eines Beschleunigungssensor-Ausgangssignals (11), das von der Atembewegung einer Brustwand (32) einer Person (30) abhängt; und
Erzeugen einer Atmungsmessungsausgabe, die sich von dem Beschleunigungssensor-Ausgangssignal unterscheidet und ein Maß für das Atemvolumen der Person bereitstellt, wobei die Atmungsmessungsausgabe unter Verwendung von maschinellem Lernen erzeugt wird und als Eingabe das Zeitdifferential des Beschleunigungssensor-Ausgangssignals verwendet.

12. Verfahren nach Anspruch 11, umfassend:
Quantifizieren des Atemvolumens der Person unter Verwendung von Kalibrierung oder Klassifizierung.

13. Computerprogramm (46), das Anweisungen enthält, die, wenn sie durch einen Prozessor ausgeführt werden, die Bestimmung einer Atmungsmessung basierend auf maschinellem Lernen veranlassen, die ein Maß für das Atemvolumen einer Person bereitstellt, wobei als Eingabe das Zeitdifferential eines empfangenen Beschleunigungssensor-Ausgangssignals (11) verwendet wird, das von der Atembewegung einer Brustwand (32) der Person (30) abhängt.

14. Computerprogramm (46) nach Anspruch 13, wobei die Anweisungen eine Quantifizierung des Atemvolumens der Person unter Verwendung einer Kalibrierung oder Klassifizierung veranlassen.

## Revendications

1. Système de mesure de volume respiratoire (100) comprenant des moyens pour :
recevoir au moins un signal de sortie de capteur d'accéléromètre (11) dépendant du mouvement respiratoire d'une paroi thoracique (32) d'un sujet (30) ; et
produire une sortie de mesure de respiration (21) différente de l'au moins un signal de sortie de capteur d'accéléromètre, qui fournit une mesure d'un volume de respiration du sujet, dans lequel la sortie de mesure de respiration est produite en utilisant l'apprentissage machine, et prend comme entrée le différentiel temporel du signal de sortie de capteur d'accéléromètre.

2. Système de mesure de volume respiratoire (100) selon la revendication 1, comprenant des moyens configurés pour quantifier le volume de respiration du sujet en utilisant un étalonnage ou une classification.

3. Système de mesure de volume respiratoire (100) selon la revendication 1 ou 2, comprenant des moyens configurés pour effectuer un apprentissage supervisé pour étalonner la sortie de mesure de respiration produite pour le sujet.

4. Système de mesure de volume respiratoire (100) selon la revendication 1 ou 2, comprenant des moyens configurés pour classer des schémas respiratoires.

5. Système de mesure de volume respiratoire (100) selon la revendication 1, 2 ou 4, comprenant des moyens configurés pour détecter une apnée du sommeil.

6. Système de mesure de volume respiratoire (100) selon l'une des revendications précédentes, dans lequel l'au moins un signal de sortie de capteur d'accéléromètre (11) comprend au moins un signal de mouvement linéaire et/ou un ou plusieurs signaux de mouvement angulaire.

7. Système de mesure de volume respiratoire (100) selon l'une des revendications précédentes, comprenant des moyens configurés pour recevoir de multiples signaux de sortie de capteur comme entrées, et pour produire une sortie de mesure de respiration qui fournit une mesure d'un volume de respiration du sujet en fonction de différentes combinaisons et/ou pondérations des multiples signaux de sortie de capteur.

8. Système de mesure de volume respiratoire (100) selon l'une des revendications précédentes, comprenant des moyens configurés pour normaliser le signal de sortie de capteur d'accéléromètre (11) pour produire un paramètre d'entrée pour un algorithme d'apprentissage machine.

9. Système de mesure de volume respiratoire (100) selon l'une des revendications précédentes, comprenant des moyens configurés pour recevoir et utiliser des signaux de sortie de capteurs d'accéléromètre provenant de multiples capteurs de mouvement (10) situés à différentes positions, pour éliminer ou atténuer les artefacts de mouvement résultant des mouvements du sujet (30) autres que les mouvements de la paroi thoracique (32) du sujet (30).

10. Système de surveillance de la santé comprenant le système de mesure respiratoire selon l'une des revendications précédentes.

11. Procédé mis en œuvre par ordinateur comprenant les étapes suivantes :
recevoir un signal de sortie de capteur d'accéléromètre (11) dépendant du mouvement respiratoire d'une paroi thoracique (32) d'un sujet (30) ; et
produire une sortie de mesure de respiration différente du signal de sortie de capteur d'accéléromètre, qui fournit une mesure d'un volume de respiration du sujet, dans lequel la sortie de mesure de respiration est produite en utilisant l'apprentissage machine, et prend comme entrée le différentiel temporel du signal de sortie de capteur d'accéléromètre.

12. Procédé selon la revendication 11 comprenant l'étape suivante :
quantifier le volume de respiration du sujet en utilisant un étalonnage ou une classification.

13. Programme informatique (46) comprenant des instructions qui, lorsqu'elles sont exécutées par un processeur, entraînent la détermination, sur la base de l'apprentissage machine, d'une mesure de respiration qui fournit une mesure d'un volume de respiration d'un sujet en utilisant comme entrée le différentiel temporel d'un signal de sortie de capteur d'accéléromètre (11) reçu dépendant du mouvement respiratoire d'une paroi thoracique (32) du sujet (30).

14. Programme informatique (46) selon la revendication 13, dans lequel les instructions entraînent la quantification du volume de respiration du sujet en utilisant un étalonnage ou une classification.
